# EUROPÄISCHE PATENTSCHRIFT

(11) **EP 2 709 571 B1**
(45) Veröffentlichungstag und Bekanntmachung des Hinweises auf die Patenterteilung: **24.02.2016**
(21) Anmeldenummer: 12730475.6
(22) Anmeldetag: 21.06.2012
(51) Int. Cl.: A61F 5/56, A61C 7/36

(54) **ZAHNAUFSATZ**
TOOTH ATTACHMENT
ACCESSOIRE DENTAIRE

(30) Priorität: 21.06.2011 DE 202011102064 U
(43) Veröffentlichungstag der Anmeldung: 26.03.2014
(73) Patentinhaber: Berk, Bianca, 35037 Marburg (DE)
(72) Erfinder: BERK, Bianca, 35037 Marburg (DE); KOEHLER, Joerg, 81245 München (DE); SAUER-SPERLING, Gotthard, 82319 Starnberg-Perchting (DE)
(74) Vertreter: Dennemeyer & Associates S.A.
(86) Internationale Anmeldenummer: PCT/EP2012/062015
(87) Internationale Veröffentlichungsnummer: WO 2012/175634

(56) Entgegenhaltungen:
- ES-A1- 2 313 843
- US-A- 6 089 864

## Beschreibung

Die vorliegende Erfindung betrifft Zahnschienen nach dem Oberbegriff des Anspruchs 1 (US 6 089 864 A), die auch Knirscherschienen genannt werden.

Im Sinne der vorliegenden Erfindung soll unter dem Begriff Knirscherschiene ganz allgemein eine Vorrichtung verstanden werden, welche geeignet ist, das Gegeneinanderpressen und Gegeneinanderreiben von Zähnen bzw. Kauflächen von Zähnen, insbesondere während des Schlafens, zurückzudrängen oder zu unterbinden bzw. die Folgen des so genannten Knirschens zu beheben oder wenigstens zu lindern.

Patienten, welche ihre Zähne zusammenpressen, werden als so genannte Knirscher bezeichnet. Die unbewussten Reibe- und Pressbewegungen der Ober- und Unterkiefer gegeneinander sind auch unter dem Begriff Bruxismus (Zähneknirschen) bekannt. Der nächtliche Schlaf-Bruxismus (so genannter nocturnaler Bruxismus) ist eine weit verbreitete Krankheit in industrialisierten Ländern. Meistens sind die Ursachen psychischer Natur. Beruflicher, privater und/oder finanzieller Stress lassen den Patienten die Zähne zusammenpressen. Dabei kann ein Druck von bis ca. 80 kg/cm² entstehen. Die dabei erzeugten Knirschgeräusche werden von den betroffenen Personen und Angehörigen meist als äußerst störend empfunden. Ein erholsamer Schlaf ist oft nicht möglich.

Auch kann Bruxismus zu Muskel-, Gesichts-, Kopf- und/oder Nackenschmerzen am darauf folgenden Tag führen. Weiterhin kann der Zahn in seiner Substanz abgeschliffen werden und sogar der Knochen kann durch den permanenten Druck, der auf ihm lastet, angegriffen werden.

Die ständige Muskelkontraktion kann zu Gefäßverengungen führen, welche wiederum Durchblutungsstörungen nach sich ziehen können. Dies kann einen Tinnitus, also Ohrengeräusche, herbeiführen. Viele Tinnituspatienten sind so genannte Knirscher.

Es ist bekannt, einem Knirscher eine maßgeschneiderte Kunststoffschiene einzusetzen. Diese verhindert jedoch lediglich den Zahnschmelzabrieb. Die oben genannten Symptome werden durch eine solche Schiene nicht verhindert.

Zudem ist bekannt, Patienten zu konditionieren. So wurde beispielsweise vorgeschlagen, die Lippen der Patienten beim Knirschen durch niedrige elektrische Impulse zu stimulieren. Der elektrische Impuls ist hierbei nicht schädlich, jedoch schmerzhaft, was nicht die Akzeptanz der Patienten findet.

Aus der ES 2 313 842 ist eine Zahnschiene bekannt, mit der zwischen senkrechtem, transversalem und intermittierendem Bruxismus unterschieden werden soll.

Der vorliegenden Erfindung liegt die Aufgabe zugrunde, eine in der Wirkung verbesserte Zahnschiene zu schaffen.

Die Aufgabe wird durch eine Zahnschiene mit den Merkmalen des Anspruchs 1 gelöst.

Die Zahnschiene umfasst hierbei eine Basiseinheit, insbesondere mit irreversibel miteinander verbundenen Zahnschienenplatten. Bevorzugt handelt es sich hierbei um einen oberen und einen unteren Teil von Zahnschienenkunststoffplatten. Die Zahnschiene umfasst zudem eine Energieversorgungseinheit, welche wiederaufladbar sein kann. Dabei kann es sich beispielsweise um eine handelsübliche Batterie oder einen handelsüblichen Akkumulator handeln.

Zudem umfasst die Zahnschiene zumindest einen Signalerzeuger, beispielsweise ein Vibrationsmodul mit einem Vibrationsmotor, und zumindest einen Drucksensor, der mit der Energieversorgungseinheit und dem Signalerzeuger in Wirkverbindung steht, wobei der Drucksensor derart ausgebildet ist, dass dieser sowohl darauf wirkende Okklusionskräfte als auch Protrusionskräfte detektieren kann.

Ferner kann zumindest eine elektrische Schaltung vorgesehen sein, die z.B. auf einer starren oder flexiblen Leiterplatte angeordnet ist. Der Drucksensor kann an die Schaltung angeschlossen werden und damit eine Wirkverbindung mit dem Signalerzeuger und der Energieversorgungseinheit eingehen.

Der oder die Drucksensoren können gegebenenfalls auch individuell an den jeweiligen Nutzer der erfindungsgemäßen Zahnschiene in Abhängigkeit eines bestimmten Schwellenwerts angepasst werden, der notwendig ist, um über den Signalerzeuger das erforderliche Signal zu generieren. Des Weiteren können auch Drucksensoren verwendet werden, die jegliches Drucksignal, sei es auch nur sehr schwach, weiterleiten. In diesem Fall kann der Signalerzeuger derart eingestellt werden, dass das gewünschte Signal, welches das Gegeneinanderpressen der Kauoberflächen der Zähne bzw. ein Gegeneinanderschieben der Vorderzähne verhindern soll, erst bei einem vorgegebenen Druckschwellenwert freigesetzt wird. Dadurch kann beispielsweise verhindert werden, dass ein Signal beim Schlucken erzeugt wird. Geeignete Drucksensoren können demgemäß für die Aktivierung bei unterschiedlichen Kaudrücken zur Verfügung gestellt werden.

Der Drucksensor ist vorzugsweise zumindest partiell eingebettet in ein Material, das kompatibel oder identisch ist mit dem Material der Basiseinheit.

Bevorzugt ist der Drucksensor zumindest partiell eingebettet in ein thermoplastisches Material und/oder ein elastomeres Material, insbesondere TPE-Material.

Bei der Zahnschiene kann es sich insbesondere um ein Kunststoffgebilde handeln, das derart geformt ist, dass es nach Art eines Gebisses in der Mundhöhle aufgenommen werden kann.

Die Zahnschiene kann beispielsweise nachträglich an das Gebiss des Trägers individuell angepasst werden. Die Zahnschiene kann auf diese Weise insbesondere eine anpassbare Zahnschiene darstellen. Alternativ kann die Zahnschiene in einem Massenherstellungsverfahren gefertigt werden, bei dem die Zahnschienen in bestimmten standardisierten Größen hergestellt werden. Die Zahnschiene ist in diesem Fall nicht individuell gefertigt.

In einem, dem Gaumen eines Patienten zugewandten Bereich können folgende elektronische Bauteile eingebaut sein: 1. Vibrationsmotor, 2. Energiegeber, z.B. Batterie, Akku, Kondensator (aufladbar, z.B. mittels Induktion oder Solar), 3. Leiterplatte, ausgestattet mit Anschlüssen zu 1. und 2. sowie den dazu gehörigen elektronischen Bauteilen. Auch ein Anschluss zu einem als Signalgeber fungierenden Drucksensor kann vorgesehen sein.

In dem Bereich zwischen den Zahnleisten der Ober- und Unterkiefer kann sich der Drucksensor befinden. Treten Okklusions- oder Protrusionskräfte auf, erfolgt beispielsweise ein Stromschluss zwischen Energieversorgungseinheit und Signalerzeuger und löst ein Signal, insbesondere eine Vibration, der gesamten Zahnschiene aus. Durch die einsetzende Vibration im sensiblen Gaumenbereich und die dadurch entstehende Geräuschentstehung im Innenohr, beispielsweise mittels Knochenleitung, kann der Patient unbewusst lernen, das Knirschen zu unterlassen. Durch das Ansprechen zwei verschiedener Sinne, hier mit Akustik und Vibration in einem Gerät, können nachgewiesenermaßen bessere Lernerfolge erzielt werden. Durch die Reize wird der unbewusste Knirschvorgang intuitiv unterbrochen. Die betroffenen Muskeln, vor allem musculus masseter, musculus temporalis und musculus medialis, werden zeit- und raumnah direkt angesprochen und mittels der Reize relaxiert. Durch diesen Mechanismus des (Bio-)Feedbacks lernt der Körper, die unerwünschte Handlung, also das Knirschen, zu unterlassen. Bei regelmäßiger Anwendung wird der Patient mittels eines negativen Reizes, z.B. Vibration, insofern konditioniert, dass er den unerwünschten Knirschvorgang unterlässt.

Die Signalerzeugung erfolgt erfindungsgemäß nicht nur beim eigentlichen Pressen und Knirschen, sondern auch beim, insbesondere häufig auftretenden, Schieben. Somit besteht auch für die Protrusion eine einfach anzuwendende Therapiemöglichkeit. Die Behandlung von, insbesondere nocturnalem Bruxismus wird dadurch verbessert.

Vorteilhafte Ausbildungen der Erfindung sind den Unteransprüchen, der Beschreibung und den Zeichnungen zu entnehmen.

Nach einer Ausführungsform kann die erfindungsgemäße Zahnschiene eine Einheit zur reversiblen Befestigung an mindestens einem Zahn, z.B. Backenzahn, oder einem Abschnitt davon darstellen.

In einer weiteren Ausführungsform stellt diese Zahnschiene eine Einheit zur reversiblen Befestigung an mindestens zwei, drei, vier oder mehr Zähnen, insbesondere Backenzähnen, dar. Die Zahnschiene kann eine Einheit zur reversiblen Befestigung an einem Eckzahn oder einem Eckzahn und mindestens einem zu diesem benachbarten Zahn oder eine Einheit zur reversiblen Befestigung an einem Eckzahn und mindestens einem, insbesondere mindestens zwei Backenzähnen, und/oder mindestens einem Eckzahn und mindestens einem, insbesondere mindestens zwei, Schneidezähnen darstellen.

Vorzugsweise stellt die erfindungsgemäße Zahnschiene eine Einheit zur reversiblen Befestigung an sämtlichen oder nahezu sämtlichen Zähnen des Ober- bzw. Unterkiefers dar.

Die Zahnschiene kann z.B. an mindestens einem, zwei, drei, vier oder mehreren ersten Backenzähnen des Ober- bzw. Unterkiefers befestigt werden.

Gemäß einer Ausführungsform umfasst der Drucksensor eine Kontakteinrichtung, die derart ausgebildet ist, dass sich deren elektrischer Widerstand abhängig von der Stärke der darauf wirkenden Okklusionskräfte und Protrusionskräfte ändert. Die Kontakteinrichtung kann beispielsweise zumindest einen elektrischen Schalter umfassen. So können z.B. leitende Drähte vorgesehen sein, die erst durch den Kaudruck in Kontakt miteinander gelangen und auf diese Weise den Signalerzeuger aktivieren. Sobald der Kaudruck wieder nachlässt, kann beispielsweise durch ein elastisches und/oder flexibles Material, in das die Drähte eingebettet sind, der Stromkreis wieder unterbrochen werden. Die Kontakteinrichtung im Sinne der vorliegenden Erfindung kann auch als Druckschalter bezeichnet werden bzw. einen solchen darstellen. Die Kontakteinrichtung kann auch Bestandteil der Basiseinheit sein oder diese bilden. Zudem ist es denkbar, zwei, drei oder mehrere Kontakteinrichtungen in der Basiseinheit vorzusehen. Häufig ist es jedoch ausreichend, nur eine einzige Kontakteinrichtung anzubringen, um den gewünschten Effekt des Zurückdrängens bzw. Unterbindens des Zähneknirschens mit der erfindungsgemäßen Zahnschiene zu erreichen. Hierbei kann auch eine einzige Kontakteinrichtung derart im Bereich der hinteren Mahl- oder Backenzähne (Molaren), im Bereich der vorderen Mahl- oder Backenzähne (Prämolaren), im Bereich der Eckzähne (Caninus) und/oder im Bereich der Front- oder Schneidezähne (Incisivi) angeordnet sein, dass sowohl Okklusions- als Protrusionskräfte detektiert werden können.

In einer Ausführungsform ist die Kontakteinrichtung entlang einer gesamten Zahnschienenplatte der erfindungsgemäßen Zahnschiene oder entlang nahezu der gesamten Ausdehnung der Zahnschienenplatte angebracht. Auf diese Weise kann sichergestellt werden, dass stets dann ein Signal ausgelöst wird, wenn durch das Zusammentreffen sich gegenüber liegender Zähne ein voreingestellter Druckwert erreicht bzw. überschritten wird.

Der Drucksensor kann auch eine Hydraulik- oder Pneumatikeinrichtung umfassen, beispielsweise in Form eines unter Druck stehenden Schlauchs. Drücken die Zähne auf den Schlauch, steigt der Druck an. Die Druckänderung kann gemessen werden, wobei ab einem bestimmten Mindestdruck ein Signalerzeuger ausgelöst wird. Dabei wird die Information über die Stärke der wirkenden Okklusions- und Protrusionskräfte durch eine Druckänderung z.B. direkt an den Signalerzeuger weitergegeben.

Die Kräfte, die zum Auslösen eines Signals erforderlich sind, können durch die Konstruktion bzw. das Material der Kontakteinrichtung eingestellt werden.

Erfindungsgemäß umfasst die Kontakteinrichtung zumindest einen Kontaktschlauch, der im Querschnitt zumindest vier Segmente aus in Umfangsrichtung abwechselnd leitendem und nichtleitendem Material, insbesondere Kunststoffmaterial, umfasst. Die Segmente des Kontaktschlauchs können sich z.B. in Längsrichtung im Wesentlichen durchgehend erstrecken. Alternativ ist auch denkbar, dass sich die Segmente nur über einen Teilabschnitt des Kontaktschlauchs erstrecken. Der Kontaktschlauch kann innen hohl sein.

Alternativ oder zusätzlich kann dieser zumindest ein elastisches und/oder flexibles Material umfassen. Dabei kann das leitende und/oder nichtleitende Material insbesondere elastisch und/oder flexibel sein, sodass dieses bei darauf wirkendem Druck zusammengedrückt werden kann. Dadurch wird ein Kontakt zwischen den leitenden Segmenten hergestellt.

Der Kontaktschlauch wird beispielsweise auf einen Anschluss der Leiterplatte gesteckt und fungiert somit als Schalter. Wird der Kontaktschlauch in einer entsprechenden Richtung komprimiert, erfolgt insbesondere ein Stromschluss zwischen Batterie und Vibrationsmotor und löst eine Vibration der gesamten Zahnschiene aus. Der Kontaktschlauch oder Sensorschlauch kann einen Längswiderstand von etwa 50 bis 180 kΩ/mm, insbesondere etwa 80 kΩ/mm besitzen. Der Isolationswiderstand ist beispielsweise größer 20 MΩ. Der Sensorschlauch kann eine Länge von beispielsweise bis zu 320 mm besitzen. Die Auslöseschwelle für den Signalerzeuger beträgt typischerweise etwa 50 kΩ.

Die Segmente sind insbesondere als im Querschnitt kreisförmiger Ring mit einem Innendurchmesser von rund einem Millimeter und einer Dicke von etwa 0,4 mm ausgebildet. Die Sektoren aus leitendem Material erstrecken sich im Querschnitt beispielsweise jeweils über etwa 100°, während sich die Segmente aus nichtleitendem Material jeweils über etwa 80° erstrecken. Der Querschnitt des Kontaktschlauches ist vorzugsweise rund, wobei auch eine andere geometrische Form, beispielsweise ein Oval, verwendet werden kann. Insbesondere kann der Kontaktschlauch in mehr als vier Segmente unterteilt sein. So sind beispielsweise auch acht Segmente aus abwechselnd leitendem und nichtleitendem Material möglich.

Gemäß einer weiteren Ausführungsform kann der Kontaktschlauch in Längserstreckung mehrere Abschnitte umfassen, wobei zumindest ein erster Abschnitt, der insbesondere im eingesetzten Zustand der Zahnschiene hinter den und/oder unterhalb der vorderen Schneidezähnen angeordnet ist, relativ zu zumindest einem zweiten Abschnitt, der insbesondere im eingesetzten Zustand der Zahnschiene an den Eckzähnen, den vorderen und/oder hinteren Backenzähnen angeordnet ist, verdreht ausgebildet ist. So kann insbesondere derselbe Kontaktschlauch zur Detektion sowohl von Okklusionskräften als auch Protrusionskräften verwendet werden.

Alternativ ist es auch möglich, mehrere Kontaktschläuche, welche zueinander verdreht sind, an der Basiseinheit anzuordnen. Ein Verdrehen innerhalb eines Kontaktschlauches ist in diesem Fall nicht notwendig. Dies ist beispielsweise auch nicht erforderlich, wenn der Kontaktschlauch mehr als vier, insbesondere acht, Segmenten umfasst.

Nach einer weiteren Ausführungsform ist zumindest ein Teil des leitenden Materials im ersten Abschnitt zu beiden Seiten einer Frontalebene angeordnet und/oder zumindest ein Teil des leitenden Materials im zweiten Abschnitt zu beiden Seiten einer Transversalebene angeordnet. Sowohl beim Schieben der Vorderzähne entlang einer Sagittalrichtung als auch beim Pressen beispielsweise der Backenzähne in Longitudinalrichtung kann auf diese Weise ein Kontakt hergestellt werden. Insbesondere kann der Kontaktschlauch im Bereich der Eckzähne sowie der vorderen und hinteren Backenzähne so verlegt sein, dass sich die elektrisch leitenden Schichten zu beiden Seiten der Transversalebene, und sich die nichtleitenden Schichten zu beiden Seiten der Frontalebene befinden. Beim klassischen Knirschvorgang, d.h. dem Pressen von oben oder unten, wird der Kontaktschlauch so komprimiert, dass sich die beiden elektrisch leitenden Schichten berühren und ein Kontakt hergestellt wird. Beim Schieben der unteren Schneidezähne nach vorne gegen die oberen Schneidezähne wird auch hier der Kontaktschlauch komprimiert, sodass ein Kontakt entsteht, da der Kontaktschlauch im Bereich hinter den vorderen Schneidezähnen um beispielsweise etwa 90° verdreht eingebaut ist, sodass sich die elektrisch leitenden Schichten zu beiden Seiten der Frontalebene und die nichtleitenden Schichten zu beiden Seiten der Transversalebene befinden.

Gemäß einer weiteren Ausführungsform umfasst der Drucksensor zumindest zwei Sensoren, die im Wesentlichen senkrecht zueinander orientiert sind.

Nach einer weiteren Ausführungsform umfasst der Signalerzeuger einen Signalgeber. Dabei kann es sich z.B. um einen mechanischen Signalgeber, einen akustischen Signalgeber, einen elektrischen Signalgeber und/oder einen thermischen Signalgeber handeln. Bei einer Kombination mehrerer Signalgeber werden beim Patienten während des Knirschens mehrere Sinne angesprochen, was die Konditionierung verbessern kann.

Der mechanische Signalgeber kann einen Vibrator bzw. ein Vibrationsmodul umfassen. Derartige Vibrationsmodule sind dem Fachmann bekannt. Beispielsweise kann es sich hierbei um solche Vibrationsmodule handeln, wie sie in Mobilfunkgeräten zum Einsatz kommen. Das Vibrationsmodul umfasst insbesondere einen Vibrationsmotor, welcher beispielsweise rotiert.

Nach einer weiteren Ausführungsform umfasst die Zahnschiene zumindest eine Speicher-, Sender- und/oder Auswerteeinheit. Dies ist insbesondere zu Diagnosezwecken hilfreich, um die gewonnenen Daten auslesen und auswerten zu können. Dabei kann beispielsweise die Leiterplatte die Speichereinheit, die Sendereinheit und/oder die Auswerteeinheit beinhalten.

Bei der Auswerteeinheit kann es sich z.B. um mindestens einen Mikroprozessor handeln, der in Wirkverbindung mit dem Drucksensor und/oder dem Signalerzeuger und/oder mindestens einer Sendereinheit steht. Durch Verwendung einer Speicher- und/oder Auswerteeinheit können die vom Drucksensor ausgehenden Signale registriert und aufbereitet werden, z.B. hinsichtlich der Häufigkeit der Signalauslösung, der Druckintensität und der Druckdauer. Mithilfe einer solchen Speicher- und/oder Auswerteeinheit ist es möglich, den Heilungsverlauf des Trägers der Zahnschiene zu verfolgen und zu dokumentieren. Die in der Speicher- und/oder Auswerteeinheit, beispielsweise einem Mikroprozessor, gespeicherten Daten können z.B. über eine separate Einheit nach Herausnahme der Zahnschiene aus dem Mund ausgelesen werden.

Alternativ oder zusätzlich kann die Sendereinheit in Wirkverbindung mit dem Drucksensor stehen. Von dem Drucksensor ausgehende Signale können über diese Sendereinheit, z.B. via Bluetooth oder Infrarot, an eine externe Empfängereinheit übermittelt und von dort an eine Speicher- und Auswerteeinheit weitergeleitet werden. Geeignete Sender- und/oder Empfangseinheiten sind dem Fachmann bekannt.

Die in der Speicher- und/oder Auswerteeinheit hinterlegten Daten können folglich über die Sendereinheit an eine entsprechende Empfängereinheit übermittelt und beispielsweise auf einem Bildschirm wiedergegeben oder gegebenenfalls ausgedruckt werden.

Gemäß einer weiteren Ausführungsform sind der Signalerzeuger und die Energieversorgungseinheit, sowie gegebenenfalls die Speicher-, Sender- und/oder Auswerteeinheit, integraler Bestandteil eines Moduls. Das Modul bildet insbesondere einen Teil der Basiseinheit oder die Basiseinheit selbst. Die Bestandteile sind insbesondere in dem Modul verbaut. Dies erleichtert die Handhabung der Zahnschiene.

Nach einer weiteren Ausführungsform ist eine Zentraleinheit, welche den Signalerzeuger und die Energieversorgungseinheit, sowie gegebenenfalls die Speicher-, Sender- und/oder Auswerteeinheit umfasst, zumindest teilweise von einer separierenden Folie umschlossen. Durch die separierende, insbesondere trennende, Folie wird ein leichtes Herauslösen aus der Schiene ermöglicht. Dadurch kann eine ordnungsgemäße und umweltverträgliche Entsorgung der Elektronikkomponenten gewährleistet werden. Auch ein Austausch und /oder eine Wiederverwendung der Zentraleinheit wird dadurch ermöglicht.

Nach einer weiteren Ausführungsform ist die Zahnschiene vollständig oder teilweise aus zumindest einem thermoplastischen Polymer und/oder zumindest einem Elastomer, z.B. TPE, gebildet.

Nach einer weiteren Ausführungsform umfasst die Energieversorgungseinheit einen wiederaufladbaren Energiespeicher. Die Aufladung kann beispielsweise mittels Induktion erfolgen. Beim Energiespeicher kann es sich z.B. um einen Lithiumionenakkumulator als Sekundärzelle, d.h. wiederaufladbaren Akkumulator, handeln. Dieser kann beispielsweise eine Nennspannung von 3,7 Volt aufweisen. Pro Batterieladung sind z.B. bis zu 2.000 Auslösungen denkbar. Insbesondere sind mehr als 500 Ladezyklen vorgesehen. Alternativ sind auch Energiespeicher denkbar, welche nicht wieder aufgeladen werden können, beispielsweise eine Primärzelle aus Lithium-Magnesium. Derartige Primärzellen können z.B. eine Nennspannung von 3 Volt haben. Hiermit sind beispielsweise bis zu 18.000 Auslösungen möglich. Die Primärzelle kann insbesondere fest in die Leiterplatte eingelötet sein.

Bei wiederaufladbaren Energiespeichern kann die Aufladung beispielsweise über das Stromnetz erfolgen. Auch der Einsatz von Solarzellen ist denkbar. So ist möglich, eine oder mehrere Solarmoduleinheiten in die erfindungsgemäße Zahnschiene und/oder in eine Ladestation zu integrieren, welche mit der Energieversorgungseinheit in Wirkverbindung steht. Beispielsweise nach Gebrauch und Reinigung kann die Zahnschiene Sonnenstrahlung ausgesetzt und somit aufgeladen werden. Es ist auch denkbar, dass ein Kondensator als Energieversorgungseinheit verwendet wird. Die Erfindung betrifft zudem eine Ladestation für eine erfindungsgemäße Zahnschiene, die als Aufbewahrungsbehältnis für die Zahnschiene ausgebildet ist. Die Ladestation ist hierbei induktiv mit der Zahnschiene gekoppelt, wodurch insbesondere ein wiederaufladbarer Energiespeicher der Zahnschiene aufladbar ist. Die Ladestation umfasst insbesondere einen induktiven Transmitter, sowie eine dazugehörige Steuerung. Die Zahnschiene umfasst hingegen eine Empfängerspule, welche vorzugsweise im Vibrationsmodul verbaut ist.

Die Fläche unter der Transmitterspule ist bevorzugt freigestellt. Beim Einbau wird die Transmitterspule z.B. schräg nach oben gebogen, um einen möglichst engen Kontakt zur Empfängerspule zu erreichen. Die Transmitterspule der Ladestation ist insbesondere als einlagige Flachspule, z.B. auf einer Leiterplattenoberseite, ausgeführt.

Die Empfängerspule der Zahnschiene kann mehrlagig und insbesondere in die Leiterplatte integriert sein. Die Spule kann beispielsweise aus etwa 50 Windungen bestehen und Teil einer Leiterstruktur der Leiterplatte sein, wobei die Fertigung insbesondere zusammen mit der restlichen Leiterplatte erfolgen kann. Insbesondere etwa 35 µm starke und 125 µm breite Leiterzüge können über sechs Lagen eines Multilayers verteilt sein. Auch die Transmitterspule der Ladestation kann als Leiterplattenspule, vorzugsweise etwa 70 µm starke und 500 µm breite Leiterzüge, ausgebildet sein. Die Abmessung der Empfängerspule im Vibrationsmodul kann z.B. ca. 25 x 20 x 0,8 mm betragen, während die Abmessung der Transmitterspule in der Ladestation etwa 30 x 28 x 1,5 mm betragen kann.

Bezüglich der Ladeposition ist ein gewisses Spiel vorhanden. So kann der Abstand zwischen den beiden Spulen während des Ladens insbesondere variabel sein und/oder beispielsweise bis ca. 8 mm betragen. Abhängig vom Abstand kann auch eine gewisse seitliche Lagetoleranz gegeben sein. So kann die seitliche Lage z.B. um einige Millimeter variieren. Innerhalb der Toleranzen wird insbesondere eine Vollladung der Sekundärzelle innerhalb von 12 Stunden ermöglicht.

Durch die Toleranz der Spulen zueinander wird der Tatsache Rechnung getragen, dass die Zahnschienen individuell an einzelne Patienten angepasst sein können, wodurch auch die Lage der Empfängerspulen in der Ladestation bei verschiedenen Zahnschienen nicht immer einheitlich ist.

Die Erfindung betrifft zudem ein Verfahren zum Herstellen einer erfindungsgemäßen Zahnschiene, umfassend die Zurverfügungstellung zumindest einer Basiseinheit, insbesondere mit irreversibel miteinander verbundenen Zahnschienenplatten, die insbesondere aus einem monolithischen Block gefräst oder gelasert sind, aus einem Material gestopft sind, aus Flüssigkunststoff gegossen sind, im Polymerisations-, Spritzguss-, Tiefzieh- oder Lasersinterverfahren, im Rapid Prototyping oder 3D-Druckverfahren, CAD/CAM-Verfahren oder Wachsverfahren hergestellt sind und/oder beispielsweise aus thermoplastischem und/oder elastomerem Material, insbesondere TPE-Material, bestehen, zumindest einer, insbesondere einen wiederaufladbaren Energiespeicher umfassenden, Energieversorgungseinheit, zumindest eines Signalerzeugers und zumindest eines Drucksensors, der mit der Energieversorgungseinheit und dem Signalerzeuger und gegebenenfalls mit einem Microprozessor in Wirkverbindung steht, und das Positionieren des Drucksensors derart, dass diese sowohl darauf wirkende Okklusionskräfte als auch Protrusionskräfte detektieren kann. Insbesondere kann bei der Herstellung ein Kontaktschlauch verdreht eingebaut werden, sodass Okklusions- und Protrusionskräfte detektiert werden können.

Bei der Zahnschiene handelt es sich insbesondere um eine Tiefziehschiene, die aus zwei Lagen Schienenfolie mit hermetisch versiegelter Zentraleinheit besteht. Diese wird im Wesentlichen nach dem gleichen Prinzip hergestellt wie normale Aufbissschienen. Der Zahnarzt nimmt hierbei einen Abdruck, z.B. mit Alginat, Impregum oder einem anderen geeigneten Material, von Ober- und Unterkiefer und sendet diesen an ein Dentallabor. Das Dentallabor fertigt aus diesem Abdruck ein Modell aus Gips. Im nächsten Schritt wird mit einem Tiefziehgerät eine Basis-Folie tiefgezogen. Auf dieser Basis-Schiene wird die Zentraleinheit platziert, welche z.B. in eine trennende und/oder isolierende Folie eingebettet ist, welche insbesondere eine ordnungsgemäße Entsorgung ermöglicht. Der Kontaktschlauch wird entsprechend dem Zahnstatus verlegt, abhängig auch davon, ob die Press- und Knirschbewegungen beim Patienten mit oder ohne Unterkieferseitenbewegungen und/oder Unterkiefervorwärtsbewegungen stattfinden.

Nach einer weiteren Ausführungsform kann zur Erhaltung der Struktur des Drucksensors, insbesondere des Kontaktschlauchs, während der Herstellung der Zahnschiene vorübergehend ein Spacer, beispielsweise ein Kunststofffaden, in den Drucksensor, z.B. in den Kontaktschlauch, eingebracht werden. Insbesondere zum Schutz des Kontaktschlauchs vor einer Kompression, z.B. während eines Tiefziehverfahrens, kann vor dem Einbau ein Spacer in diesen, insbesondere im Querschnitt zentral, eingeführt werden. Der Spacer kann sich über die gesamte Länge des Kontaktschlauchs erstrecken.

Zur Versiegelung der elektronischen Teile und/oder des Drucksensors, insbesondere des Kontaktschlauchs, wird, insbesondere nach der Platzierung auf der Basis-Schiene, z.B. eine zweite Folie über die Basis-Schiene, die elektronischen Teile und/oder den Drucksensor, insbesondere den Kontaktschlauch, tiefgezogen. Danach wird der Spacer wieder aus dem Kontaktschlauch herausgezogen und das entstandene Loch in der Folie wieder viersiegelt. Somit kann insbesondere erreicht werden, dass der Kontaktschlauch innen hohl ist. Dies ist wünschenswert, da der Schlauch somit bei Druckbeaufschlagung durch die Zähne seine Form ändern und komprimiert werden kann.

Eine Zentraleinheit, welche den Signalerzeuger und die Energieversorgungseinheit, sowie gegebenenfalls die Speicher-, Sender- und/oder Auswerteeinheit umfasst, kann während der Herstellung der Zahnschiene zumindest teilweise von einer separierenden Folie aus einem geeigneten Material umschlossen werden.

Zudem ist ein Verfahren zum Betreiben einer, insbesondere erfindungsgemäßen, Zahnschiene offenbart, das umfasst: das Detektieren der Stärke von wirkenden Okklusionskräften und Protrusionskräften mithilfe eines Drucksensors, insbesondere einer als Kontaktschlauch ausgebildeten Kontakteinrichtung, deren elektrischer Widerstand sich abhängig von der Stärke der darauf wirkenden Okklusionskräfte und Protrusionskräfte ändert, die mittelbare oder unmittelbare Weitergabe der Stärke-Information an einen Signalerzeuger, die Erzeugung eines Signals, durch den Signalerzeuger, wenn die Stärke einen vorbestimmten Grenzwert überschreitet, und das Aussetzen des Signals, wenn die Stärke den vorbestimmten Grenzwert unterschreitet, wobei der Drucksensor und der Signalerzeuger von einer Energieversorgungseinheit mit Energie versorgt werden, welche insbesondere einen wiederaufladbaren Energiespeicher umfasst.

Nachfolgend wird die vorliegende Erfindung rein beispielhaft anhand vorteilhafter Ausführungsformen unter Bezugnahme auf die beigefügten Zeichnungen beschrieben. Es zeigen:
- Fig. 1: eine Draufsicht einer Ausführungsform einer erfindungsgemäßen Zahnschiene,
- Fig. 2: eine Schnittansicht einer ersten Ausführungsform eines erfindungsgemäßen Kontaktschlauchs,
- Fig. 3: eine Schnittansicht einer zweiten Ausführungsform eines erfindungsgemäßen Kontaktschlauchs,
- Fig. 4: eine Schnittansicht gemäß Fig. 1 (A - B),
- Fig. 5: eine Schnittansicht gemäß Fig. 1 (C - D),
- Fig. 6a - 6c: verschiedene Ansichten einer Zentraleinheit mit Primärzelle einer erfindungsgemäßen Zahnschiene,
- Fig. 7a - 7c: verschiedene Ansichten einer Zentraleinheit mit Sekundärzelle einer erfindungsgemäßen Zahnschiene,
- Fig. 8: ein Diagramm zur Darstellung einer Auslöseschwelle,
- Fig. 9: ein Blockschaltbild einer Zentraleinheit einer erfindungsgemäßen Zahnschiene,
- Fig. 10a - 10c: verschiedene Ansichten einer Ladestation einer erfindungsgemäßen Zahnschiene, und
- Fig. 11: ein Blockschaltbild einer Ladestation einer erfindungsgemäßen Zahnschiene.

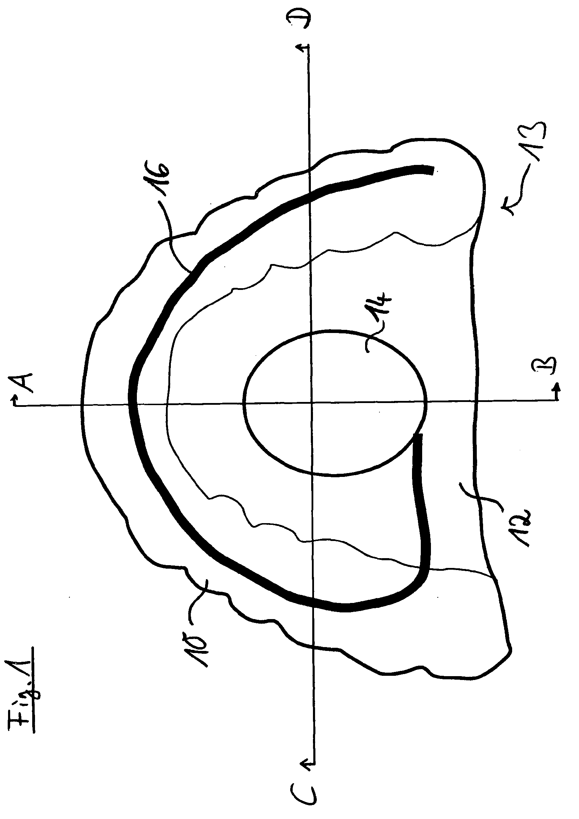

Fig. 1 zeigt eine Zahnschiene mit einem Zahnbogen 10 und einem Gaumenteil 12. Der Zahnbogen 10 und das Gaumenteil 12 bilden zusammen eine Basiseinheit 13. Am Gaumenteil 12 ist eine Zentraleinheit 14 angeordnet, welches beispielsweise eine Elektronik mit einem Vibrationsmotor und einer Energieversorgungseinheit umfassen kann.

Im Bereich des Zahnbogens 10 ist ein Drucksensor als Kontakteinrichtung angeordnet, welcher in diesem Ausführungsbeispiel als Kontaktschlauch 16 ausgebildet ist. Der Kontaktschlauch 16 steht mit der Zentraleinheit 14 in Wirkverbindung und ist insbesondere elektrisch mit dieser verbunden.

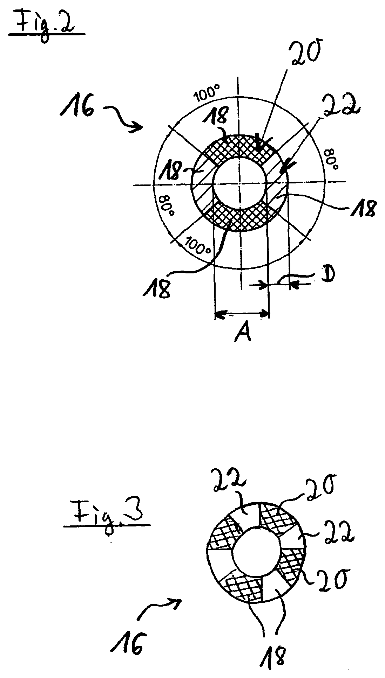

Fig. 2 zeigt eine Schnittansicht des Kontaktschlauchs 16. Dieser ist im Querschnitt in vier Kompartimente oder Segmente 18, unterteilt. Die Segmente oder Sektoren sind jeweils abwechselnd aus leitendem Material 20 und nichtleitendem Material 22 gebildet. Der Innendurchmesser A kann beispielsweise 1 mm betragen, während die Dicke D ca. 0,4 mm betragen kann. Das nichtleitende Material 22 wirkt isolierend und umfasst insbesondere ein flexibles Material. Dieses kann somit bei auftretenden Okklusions- und Protrusionskräften komprimiert werden, sodass sich die beiden Segmente 18 mit leitendem Material 20 berühren und somit einen elektrischen Kontakt herstellen können. Dadurch ändert sich der elektrische Widerstand des Kontaktschlauchs 16, der insbesondere innen hohl ist.

In Fig. 3 ist eine weitere Ausführungsform eines Kontaktschlauchs 16 gezeigt. Dieser umfasst acht Segmente 18 und somit vier Segmente 18 aus leitendem Material 20 und vier aus nichtleitendem Material 22.

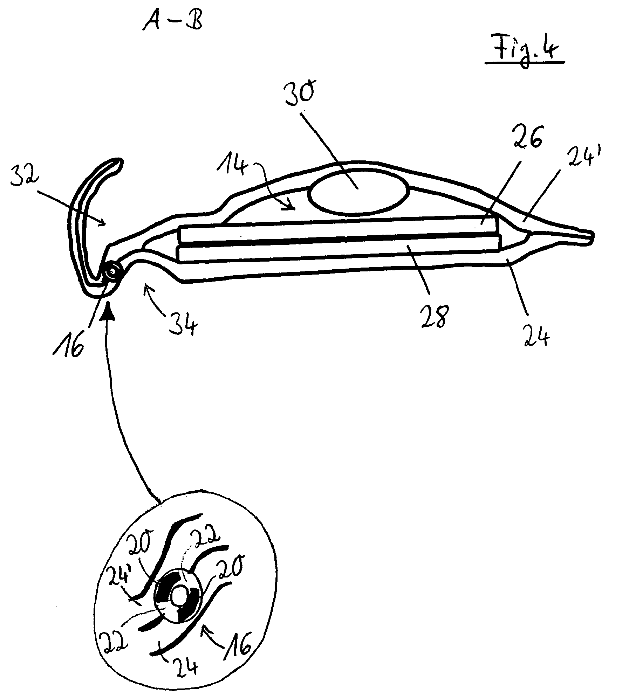

Fig. 4 zeigt eine Schnittansicht gemäß der Linie A - B von Fig. 1. Zwischen einer Tiefziehfolie 24', welche an der Gaumenseite angeordnet ist, und einer Tiefziehfolie 24, welche an der Zungenseite angeordnet ist, befindet sich die Zentraleinheit 14. Dieses umfasst eine Energieversorgungseinheit 26 mit einem Energiespeicher, welcher als Batterie oder als, insbesondere wiederaufladbarer, Akkumulator ausgebildet sein kann. Zudem kann die Zentraleinheit 14 eine Elektronik 28 und einen Signalerzeuger umfassen, welcher in diesem Ausführungsbeispiel als Vibrationsmodul 30 ausgebildet ist. Das Vibrationsmodul 30 umfasst z.B. einen insbesondere rotierenden Vibrationsmotor. Alternativ ist es auch möglich, die Elektronik 28 wegzulassen und den Vibrationsmotor direkt mit dem Drucksensor und der Energiequelle zu schalten.

Zwischen dem Bereich der oberen Schneidezähne 32 und dem Bereich der unteren Schneidezähne 34 ist ein Kontaktschlauch 16 angeordnet. Je nach individuellem Zahnstatus kann der Kontaktschlauch auch höher oder tiefer als in der Zeichnung dargestellt verlegt werden. Ein entsprechender Ausschnitt ist zusammen mit den Tiefziehfolien 24, 24' in Fig. 4 auch vergrößert dargestellt. Die Segmente 18 mit leitendem Material 20 sind hierbei seitlich angeordnet, sodass bei einer seitlichen Bewegung der Schneidezähne, d.h. einer Protrusion, ein elektrischer Kontakt hergestellt werden kann. Der Kontaktschlauch 16 entspricht der Ausführungsform gemäß Fig. 2. Alternativ ist auch denkbar, beispielsweise einen Kontaktschlauch 16 gemäß Fig. 3 zu verwenden. Der Kontaktschlauch 16 wird derart positioniert, dass bei einer Protrusion ein elektrischer Kontakt zwischen zwei leitenden Materialen 20 hergestellt wird.

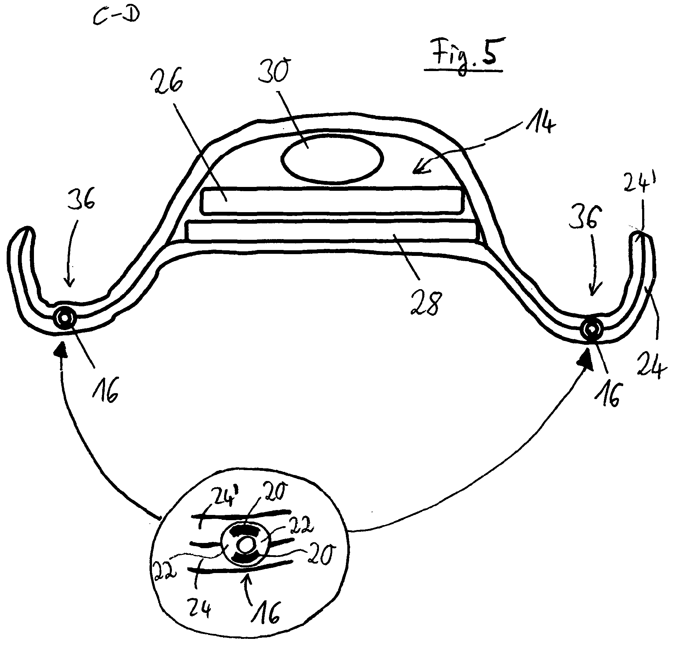

In Fig. 5 ist eine Schnittansicht gemäß der Linie C - D von Fig. 1 gezeigt. Der Kontaktschlauch 16 ist hierbei an einem Bereich der oberen Backenzähne 36 angeordnet. Das leitende Material 20 befindet sich oben und unten im Kontaktschlauch 16 (siehe vergrößerte Darstellung). Bei Okklusionskräften wird somit ein elektrischer Kontakt zwischen den leitenden Materialien 20 hergestellt.

Der Kontaktschlauch 16 ist im Bereich der Schneidezähne 32, 34 (siehe Fig. 4) im Vergleich zum Bereich der oberen Backenzähne 36 (siehe Fig. 5) verdreht. Wird beispielsweise ein Kontaktschlauch 16 gemäß Fig. 3 verwendet, ist hingegen eine Verdrehung des Kontaktschlauchs 16 nicht unbedingt notwendig. In beiden Fällen ist es somit möglich, mithilfe eines einzigen Kontaktschlauchs 16 sowohl Okklusions- als auch Protrusionskräfte detektieren zu können.

Ab einem bestimmten Druck wird ein Signal an das Vibrationsmodul 30 übertragen. Eine Vibration wird daraufhin ausgelöst, wodurch der Patient aufhört, mit den Zähnen zu knirschen.

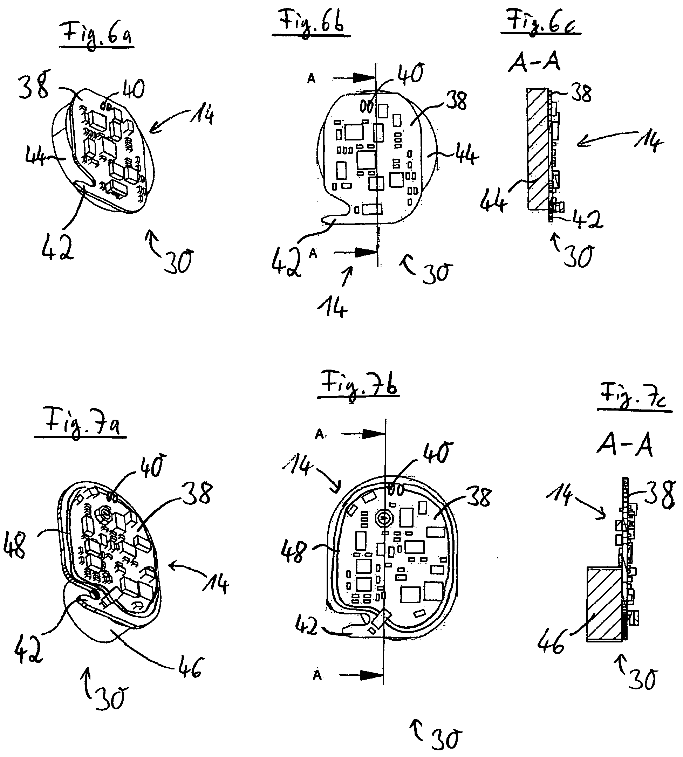

Verschiedene Ansichten eines Ausführungsbeispiels der Zentraleinheit 14 sind in den Fig. 6a, Fig. 6b und Fig. 6c dargestellt. Die Zentraleinheit 14 umfasst eine Leiterplatte 38 mit einem Motoranschluss 40. Der Motor ist von der Leiterplatte 38 abgesetzt und daher nicht abgebildet. Zudem umfasst die Leiterplatte 38 einen Anschluss 42 für einen Kontaktschlauch 16. Der Anschluss 42 ist z.B. als partielle Rand-Verkupferung der Leiterplatte 38 ausgeführt. Eine optionale Vergoldung verhindert eine Oxidation bei der Lagerung und im Betrieb. Die Leiterplatte 38 ist an einer Primärzelle 44 angeordnet. Es ist auch möglich, die Zentraleinheit 14 und den Motor mit Hilfe einer gemeinsamen Starr-Flex-Leiterplatte zu verbinden, um die Anschlussdrähte am Motor zu ersetzen.

Die Zentraleinheit 14 gemäß Fig. 7a, Fig. 7b und Fig. 7c entspricht im Wesentlichen der Zentraleinheit 14 gemäß Fig. 6. Die Leiterplatte 38 ist in dieser Ausführungsform jedoch an einer Sekundärzelle 46 angeordnet. Die Zentraleinheit 14 verfügt somit über eine wiederaufladbare Energiequelle. Zum Aufladen kann die Leiterplatte 38 beispielsweise über eine Empfängerspule 48 verfügen.

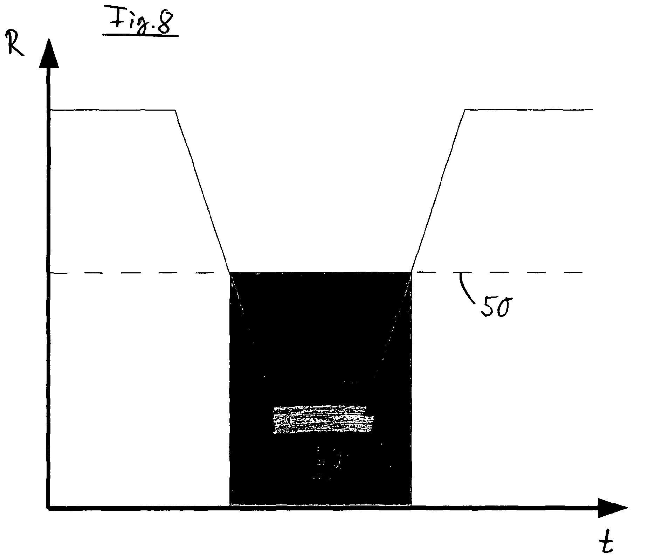

Fig. 8 zeigt ein Diagramm zur Darstellung der Aktivierung einer Vibration. Hierbei ist der elektrische Widerstand R gegen die Zeit t aufgetragen. Die gestrichelte Linie stellt eine Auslöseschwelle 50 bzw. einen Grenzwert dar, während der grau hinterlegte Bereich einen Vibrationsbereich 52 definiert. Zur Aktivierung der Vibration wird ein Kontaktschlauch 16 verwendet, dessen durch den Druck der Zähne ausgelöste Widerstandsänderung in der Zentraleinheit 14 ausgewertet wird. Dabei vergleicht ein Komparator das Sensorsignal mit einer festen Schwelle 50. Durch den Druck auf den Kontaktschlauch 16 wird ein elektrischer Kontakt zwischen den leitenden Materialien 20 hergestellt, wodurch der Widerstand R im Kontaktschlauch 16 sinkt. Wird die Auslöseschwelle 50 unterschritten, schaltet sich der Vibrationsmotor des Vibrationsmoduls 30 ein und vibriert. Durch die Vibration löst sich der Kiefer, der Druck auf den Kontaktschlauch sinkt, wodurch der elektrische Widerstand R im Kontaktschlauch 16 ansteigt. Die Vibration wird wieder unterbunden.

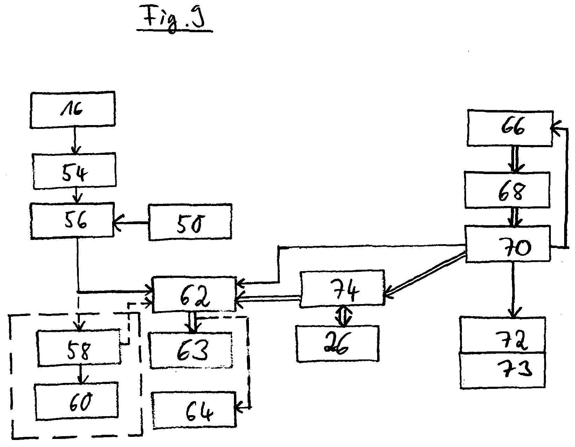

Fig. 9 zeigt ein Blockschaltbild einer Zentraleinheit 14. Einfache Pfeile repräsentieren hierbei ein übertragenes Signal, während Doppelpfeile die Energie symbolisieren. Ein Drucksensor, beispielsweise ein Kontaktschlauch 16, detektiert zunächst die Stärke der Okklusion bzw. Protrusion. Das Signal wird in einem Filter 54 gefiltert und gelangt zu einem Komparator 56. Dort wird das Signal mit einer Auslöseschwelle 50 verglichen. Optional wird das Signal in einen Vibrationskontrollspeicher 58 und eine optische Datenübertragungseinheit 60 geleitet. Über einen DC/DC-Wandler 62 gelangt Energie in einen Vibrationsmotor 63. Dass die Zentraleinheit 14 aktiv ist, kann beispielsweise durch eine Anzeige 64 angezeigt werden. Wird eine wiederaufladbare Batterie bzw. ein Akkumulator verwendet, kann zudem ein Übertrager 66 vorgesehen sein, der Energie in einen Gleichrichter 68 und einen Laderegler 70 leitet. Der Ladezustand kann durch einen Ladeanzeiger 72 angezeigt werden. Ein Ladefeedback 73 kann hierbei ausgegeben werden. Der Laderegler 70 sowie eine Batterie bzw. ein Akkumulator als Energieversorgungseinheit 26 sind mit einem Lade- bzw. Entladeschutz 74 verbunden. Der DC/DC-Wandler 62 wird auf diese Weise mit Energie versorgt.

Das Energiespannungsniveau wird mithilfe des Gleichspannungswandlers 62 auf die Betriebsspannung des Vibrationsmotors 63 reduziert. Bei Einsatz einer Primärzelle 44 wird der Gleichspannungswandler 62 durch einen Schalter ersetzt, da die Betriebsspannungsniveaus in diesem Fall ähnlich liegen.

Ein unabhängiger Lade- und/oder Entladeschutz 74 überwacht die Batteriespannung und/oder den Batteriestrom und verhindert gegebenenfalls weiteres Auf- und/oder Entladen. Diese Funktion wird nur bei Einsatz einer wiederaufladbaren Batterie, d.h. Sekundärzelle 46, vollständig bestückt. Bei einer Primärzelle 44 kommt eine, insbesondere selbstrückstellende, Sicherung zum Einsatz.

Die Energieübertragung beim Aufladen erfolgt insbesondere induktiv mithilfe einer in die Leiterplatte 38 integrierten Spule (siehe Fig. 7a - c), die zusammen mit einer Kapazität auf die Übertragungsfrequenz abgestimmt ist. Die gleichgerichtete Wechselspannung wird über den Laderegler 70, insbesondere einen Shuntregler, zur Ladung der Sekundärzelle 46 genutzt. Mithilfe einer Feedbackschaltung kann die Aktivierung bzw. Deaktivierung der Ladefunktion und/oder der Ladezustand an das Lademodul 76 übertragen werden, um zum Ladeende die weitere Energieübertragung auf das Modul zu verhindern. Das Feedback dient gleichzeitig als Schutzfunktion, um eine unzulässig hohe Erwärmung von, insbesondere metallischen, Fremdkörpern, die über der Spule des Lademoduls platziert werden, zu verhindern. Der Ladeanzeiger 72 wird aktiviert, nachdem die induktive Energieübertragung aktiv wird und sich die Batteriespannung im Ladebereich bewegt.

Eine Vibrationskontrolle, z.B. in Form eines Mikrocontrollers und passender Software, mit einem Vibrationskontrollspeicher 58, z.B. für die Anzahl und Dauer der Vibrationen, kann optional integriert werden. Auf diese Weise kann ein Nutzungsprofil zur Bewertung des Therapieerfolgs erstellt werden. Die Ansteuerung des Vibrationsmotors 63 erfolgt dann durch die Vibrationskontrolle anstatt direkt durch den Komparator 56. Die gespeicherten Daten können über eine optische Schnittstelle, d.h. mithilfe einer optischen Datenübertragungseinheit 60, seriell ausgelesen werden.

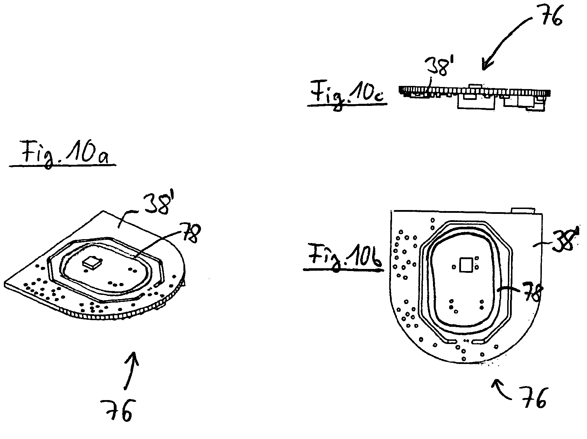

Die Fig. 10a, Fig. 10b und Fig. 10c zeigen jeweils unterschiedliche Ansichten eines Lademoduls 76 bzw. einer Ladestation 76. Diese kann beispielsweise in ein als Transportbox ausgebildetes Aufbewahrungsbehältnis eingebaut sein. Das Lademodul 76 umfasst eine Leiterplatte 38' mit einer Transmitterspule 78. Diese ist freigestellt, d.h. die Leiterplatte 38' ist innen geschlitzt, und wird beim Einbau um die Drehachse schräg nach oben gebogen, um einen möglichst engen Kontakt zur Empfängerspule 48 des Vibrationsmoduls 30 zu erreichen (siehe Fig. 7a - c). Die Leiterplatte 38 ist im Bereich der Drehachse einseitig tiefengefräst, weshalb die elektrischen Verbindungen zu einem Photosensor und zur Transmitterspule 78 nur auf der nicht gefrästen Seite verlaufen können. Die Endlage, d.h. der Kippwinkel, kann an die konkrete Einbausituation und Baugröße des Vibrationsmoduls 30 angepasst werden. Die Transmitterspule 78 des Lademoduls 76 ist insbesondere als einlagige Flachspule ausgeführt.

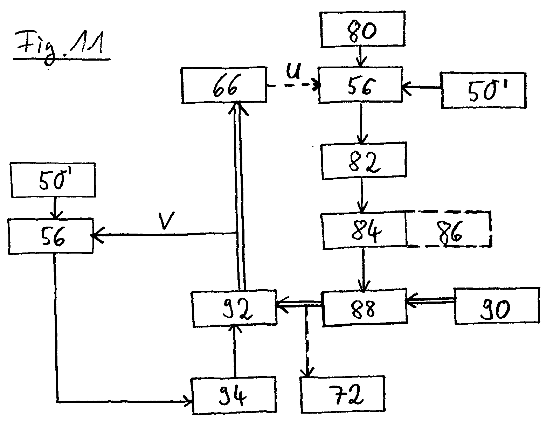

Fig. 11 zeigt ein Blockschaltbild eines Lademoduls 76. Einfache Pfeile stellen hierbei ein Signal dar, während Doppelpfeile die Energie repräsentieren. Fließender Strom wird durch ein V dargestellt, während die Spannung durch ein U symbolisiert wird. Gestrichelte Linien stellen jeweils optionale Bauteile dar. Das Signal eines Photoempfängers 80 wird von einem Komparator 56 mit einem Grenzwert als Schwelle 50' verglichen. Ein Monoflop 82, der insbesondere retriggerbar ist, wird aktiviert. Das Signal wird an eine Pulsweitenmodulation (PWM) 84 weitergegeben, welche optional über eine Ladekontrolle 86 verfügt. Daraufhin wird ein Schalter 88 aktiviert, der über ein Steckernetzteil 90 mit Energie versorgt wird. Ein Ladeanzeiger 72 gibt optional den Ladezustand aus. Energie wird an einen Treiber 92, insbesondere eine Halbbrücke, weitergegeben, wodurch ein Übertrager 66, insbesondere ein Resonanzkreis, mit Energie versorgt wird. Der Treiber 92 erhält ein Signal eines Oszillators 94. Dieser Oszillator 94 erhält, außer bei Überstrom, ein Signal eines Komparators 56, welcher den Strom V mit einem Grenzwert als Schwelle 50' vergleicht.

Die Energieübertragung vom Lademodul 76 zur Zentraleinheit 14 erfolgt durch induktive Kopplung zweier Spulen im Transmitter und Empfänger. Die Übertrager 66 im Lademodul 76 und in der Zentraleinheit 14 werden dabei in Resonanz betrieben. Um eine ungewollte Übertragung von Energie auf andere Objekte außer der Zentraleinheit 14 oder nach dem Ende der Ladung zu verhindern, erfolgt zwischen dem Lademodul 76 und der Zentraleinheit 14 ein Handshake.

Ein Oszillator 94 erzeugt im Standby-Modus unabhängig von der Anwesenheit eines Empfängers ein zyklisches Aktivierungssignal und schließt den Schalter 88 zur Energieversorgung 90 der Ladeschaltung, z.B. für 100 ms alle zwei Sekunden, was einer Einschaltzeit von 5 % entspricht.

In der Zentraleinheit 14 wird bei Aktivierung der internen Stromversorgung ein Feedbacksignal erzeugt, insbesondere mithilfe einer LED. Dieses Ladefeedbacksignal wird im Lademodul 76 mithilfe eins Photoempfängers 80, insbesondere einer Photodiode, erfasst und einem Komparator 56 zugeführt. Bei Überschreiten einer Schwelle 50' wird die korrekte Energieversorgung zur Zentraleinheit 14 erkannt und die Aktivierungszeit entsprechend erhöht, z.B. auf eine Einschaltzeit von 95 %.

Um die Anwesenheit der Zentraleinheit 14 weiterhin erkennen zu können, erfolgt im Lade-Modus eine zyklische Abschaltung der Energieübertragung, z.B. für 100 ms alle zwei Sekunden, was einer Einschaltzeit von 95 % und einer Abschaltzeit von 5 % entspricht. Bleibt bei der nächsten Aktivierung das optische Feedbacksignal der Zentraleinheit 14 aus, wird die Energieübertragung nach einigen Sekunden wieder auf den Standby-Modus mit 5 % Einschaltzeit zurückgeschaltet. Auch bei Erreichen des Ladeendes wird das Feedbacksignal nicht mehr aktiviert, sodass keine weitere Energieübertragung über die induktive Schnittstelle erfolgt.

Die Erhöhung der Aktivierungszeit wird dabei von einem Monoflop 82 gesteuert, der zyklisch reaktiviert werden muss. Bleiben die Aktivierungsimpulse aus, z.B. weil die Zentraleinheit 14 entfernt oder die Energieversorgungseinheit 26 vollständig geladen ist, wird der Monoflop 82 nicht mehr nachgetriggert und schaltet nach seiner Zykluszeit wieder in den Standby-Modus.

Die Steuerung der Ein- und Ausschaltzeiten erfolgt mithilfe einer PWM-Stufe 84. Die Steuerspannung wird dabei vom Zustand des Monoflops 82 bestimmt und legt das PWM-Verhältnis auf 5 % bzw. 95 % fest. Eine Anpassung an andere Werte ist leicht möglich.

Der in der Spule 78 der induktiven Energieübertragung fließende Strom V wird mithilfe eines Komparators 56 überwacht. Bei Überschreiten einer Schwelle 50' wird der Oszillator 94 gestoppt und die Energieübertragung unterbrochen. Somit ist eine Sicherung gegeben.

Die Energieversorgung des Lademoduls 76 erfolgt über ein Steckernetzteil 90 oder z.B. einen USB-Anschluss eines PCs. Optional kann eine Mikrocontroller-gesteuerte Ladekontrolle bzw. ein Spannungsfeedback der Transmitterspule 78 integriert werden.

Standardmäßig sind sowohl die Zentraleinheit 14 als auch das Lademodul 76 nicht mit einem Mikrocontroller und entsprechender Software ausgestattet. Es besteht jedoch die Möglichkeit, bei beiden jeweils als Bestückungsvariante optional einen Mikrocontroller nachzurüsten, um beispielsweise Nutzungsprofile, z.B. Veränderung des Klientenverhaltens, zu sammeln, Vibrationsmuster zu steuern oder um komplexere Ladeverfahren zu implementieren.

### Bezugszeichenliste

- 10: Zahnbogen
- 12: Gaumenteil
- 13: Basiseinheit
- 14: Zentraleinheit
- 16: Drucksensor, Kontaktschlauch
- 18: Segment
- 20: leitendes Material
- 22: nichtleitendes Material
- 24, 24': Tiefziehfolie
- 26: Energieversorgungseinheit
- 28: Elektronik
- 30: Signalerzeuger, Vibrationsmodul
- 32: Bereich der oberen Schneidezähne
- 34: Bereich der unteren Schneidezähne
- 36: Bereich der oberen Backenzähne
- 38, 38': Leiterplatte
- 40: Motoranschluss
- 42: Anschluss
- 44: Primärzelle
- 46: Sekundärzelle
- 48: Empfängerspule
- 50, 50': Schwelle
- 52: Vibrationsbereich
- 54: Filter
- 56: Komparator
- 58: Vibrationskontrollspeicher
- 60: optische Datenübertragungseinheit
- 62: DC/DC-Wandler, Gleichspannungswandler
- 63: Vibrationsmotor
- 64: Anzeige
- 66: Übertrager
- 68: Gleichrichter
- 70: Laderegler
- 72: Ladeanzeiger
- 73: Ladefeedback
- 74: Lade-, Entladeschutz
- 76: Lademodul, Ladestation
- 78: Transmitterspule
- 80: Photoempfänger
- 82: Monoflop
- 84: Pulsweitenmodulation (PWM)
- 86: Ladekontrolle
- 88: Schalter
- 90: Steckernetzteil, Energieversorgung
- 92: Treiber
- 94: Oszillator

- A: Innendurchmesser
- D: Dicke
- R: elektrischer Widerstand
- t: Zeit
- V: Strom
- U: Spannung

## Patentansprüche

1. Zahnschiene, umfassend
- eine Basiseinheit (13), insbesondere mit irreversibel miteinander verbundenen Zahnschienenplatten,
- zumindest eine Energieversorgungseinheit (26),
- zumindest einen Signalerzeuger (30), und
- zumindest einen Drucksensor (16), der mit der Energieversorgungseinheit (26) und dem Signalerzeuger (30) in Wirkverbindung steht, und der eine Kontakteinrichtung mit zumindest einem elektrischen Schaltkontakt umfasst,
wobei der Drucksensor (16) derart ausgebildet ist, dass dieser sowohl darauf wirkende Okklusionskräfte als auch Protrusionskräfte detektieren kann,
**dadurch gekennzeichnet, dass**
die Kontakteinrichtung zumindest einen Kontaktschlauch (16) umfasst, der im Querschnitt zumindest vier Segmente (18) aus in Umfangsrichtung abwechselnd leitendem (20) und nichtleitendem Material (22) umfasst.

2. Zahnschiene nach Anspruch 1,
**dadurch gekennzeichnet, dass**
das Material Kunststoffmaterial ist.

3. Zahnschiene nach Anspruch 1 oder 2,
**dadurch gekennzeichnet, dass**
sich die Segmente (18) des Kontaktschlauchs (16) in Längsrichtung im Wesentlichen durchgehend erstrecken.

4. Zahnschiene nach einem der Ansprüche 1 bis 3,
**dadurch gekennzeichnet, dass**
der Kontaktschlauch (16) innen hohl ist.

5. Zahnschiene nach einem der Ansprüche 1 bis 4,
**dadurch gekennzeichnet, dass**
der Kontaktschlauch (16) in Längserstreckung mehrere Abschnitte umfasst, wobei zumindest ein erster Abschnitt, der insbesondere im eingesetzten Zustand der Zahnschiene hinter den und/oder unterhalb der vorderen Schneidezähnen angeordnet ist, relativ zu zumindest einem zweiten Abschnitt, der insbesondere im eingesetzten Zustand der Zahnschiene an den Eckzähnen, den vorderen und/oder hinteren Backenzähnen angeordnet ist, verdreht ausgebildet ist.

6. Zahnschiene nach Anspruch 5,
**dadurch gekennzeichnet, dass**
zumindest ein Teil des leitenden Materials (20) im ersten Abschnitt zu beiden Seiten einer Frontalebene angeordnet ist und dass zumindest ein Teil des leitenden Materials (20) im zweiten Abschnitt zu beiden Seiten einer Transversalebene angeordnet ist.

7. Zahnschiene nach einem der vorstehenden Ansprüche,
**dadurch gekennzeichnet, dass**
der Drucksensor (16) zumindest zwei Sensoren umfasst, die im Wesentlichen senkrecht zueinander orientiert sind.

8. Zahnschiene nach einem der vorstehenden Ansprüche,
**dadurch gekennzeichnet, dass**
der Signalerzeuger (30) und die Energieversorgungseinheit (26), sowie gegebenenfalls eine Speicher-, Sender- und/oder Auswerteeinheit, integraler Bestandteil eines Moduls sind.

9. Zahnschiene nach Anspruch 8,
**dadurch gekennzeichnet, dass**
das Modul einen Teil der Basiseinheit (13) oder die Basiseinheit (13) selbst bildet.

10. Zahnschiene nach einem der vorstehenden Ansprüche,
**dadurch gekennzeichnet, dass**
eine Zentraleinheit (14), welche den Signalerzeuger (30) und die Energieversorgungseinheit (26), sowie gegebenenfalls eine Speicher-, Sender- und/oder Auswerteeinheit umfasst, zumindest teilweise von einer separierenden Folie umschlossen ist.

11. Zahnschiene nach einem der vorstehenden Ansprüche,
**dadurch gekennzeichnet, dass**
diese vollständig oder teilweise aus zumindest einem thermoplastischen Polymer oder zumindest einem Elastomer gebildet ist.

12. Ladestation (76) für eine Zahnschiene nach einem der vorhergehenden Ansprüche, die als Aufbewahrungsbehältnis für die Zahnschiene ausgebildet ist, wobei die Ladestation (76) induktiv mit der Zahnschiene gekoppelt ist, wodurch ein wiederaufladbarer Energiespeicher der Zahnschiene aufladbar ist.

13. Verfahren zum Herstellen einer Zahnschiene nach einem der vorhergehenden Ansprüche, umfassend
die Zurverfügungstellung
- zumindest einer Basiseinheit (13), insbesondere mit irreversibel miteinander verbundenen Zahnschienenplatten, die insbesondere aus einem monolithischen Block gefräst oder gelasert sind, aus einem Material gestopft sind, aus Flüssigkunststoff gegossen sind, im Polymerisations-, Spritzguss-, Tiefzieh- oder Lasersinterverfahren, im Rapid Prototyping, 3D-Druckverfahren, CAD/CAM-Verfahren oder Wachsverfahren hergestellt sind und/oder beispielsweise aus thermoplastischem und/oder elastomerem Material, insbesondere TPE-Material, bestehen,
- zumindest einer, insbesondere einen wiederaufladbaren Energiespeicher umfassenden, Energieversorgungseinheit (26),
- zumindest eines Signalerzeugers (30) und
- zumindest eines Drucksensors (16), der mit der Energieversorgungseinheit (26) und dem Signalerzeuger (30) in Wirkverbindung steht, und
das Positionieren des Drucksensors (16) derart, dass dieser sowohl darauf wirkende Okklusionskräfte als auch Protrusionskräfte detektieren kann.

14. Verfahren nach Anspruch 13,
**dadurch gekennzeichnet, dass**
zur Erhaltung der Struktur des Drucksensors (16) während der Herstellung der Zahnschiene vorübergehend ein Spacer in den Drucksensor (16) eingebracht wird.

## Claims

1. A dental splint, comprising
- a base unit (13), especially one with dental splint plates irreversibly connected to one other;
- at least one power supply unit (26);
- at least one signal generator (30); and
- at least one pressure sensor (16) which is operatively connected to the power supply unit (26) and to the signal generator (30) and which comprises a contact device having at least one electrical switch contact,
wherein the pressure sensor (16) is configured such that it can detect both occlusion forces acting on it and protrusion forces,
**characterized in that**
the contact device comprises at least one contact sleeve (16), which comprises in cross section at least four segments (18) of conductive (20) and nonconductive material (22) alternating in the circumferential direction.

2. A dental splint according to claim 1,
**characterized in that**
the material is a plastic material.

3. A dental splint according to claim 1 or claim 2,
**characterized in that**
the segments (18) of the contact sleeve (16) essentially extend continuously in the longitudinal direction.

4. A dental splint according to any one of the claims 1 to 3,
**characterized in that**
the contact sleeve (16) is hollow on the inside.

5. A dental splint according to any one of the claims 1 to 4,
**characterized in that**
the contact sleeve (16) comprises a plurality of sections in a longitudinal extent, wherein at least one first section, which in the installed condition of the dental splint is in particular arranged behind and/or beneath the front incisors, is twisted relative to at least one second section, which in the installed condition of the dental splint is in particular arranged at the cuspids, at the premolars and/or at the molars.

6. A dental splint according to claim 5,
**characterized in that**
at least some of the conductive material (20) in the first section is arranged at both sides of a frontal plane and **in that** at least some of the conductive material (20) in the second section is arranged at both sides of a transverse plane.

7. A dental splint according to any one of the preceding claims,
**characterized in that**
the pressure sensor (16) comprises at least two sensors which are oriented essentially perpendicular to one other.

8. A dental splint according to any one of the preceding claims,
**characterized in that**
the signal generator (30) and the power supply unit (26) and also optionally a storage unit and a transmitter and/or evaluation unit are integral components of a module.

9. A dental splint according to claim 8,
**characterized in that**
the module forms a part of the base unit (13), or itself forms the base unit (13).

10. A dental splint according to any one of the preceding claims,
**characterized in that**
a central unit (14) which comprises the signal generator (30) and the power supply unit (26), and also optionally a storage unit, and a transmitter and/or evaluation unit is at least partly enclosed by a separating film.

11. A dental splint according to any one of the preceding claims,
**characterized in that**
it is formed entirely or partly from at least one thermoplastic polymer and/or at least one elastomer.

12. A charging station (76) for a dental splint according to any one of the preceding claims, which is configured as a storage receptacle for the dental splint, wherein the charging station (76) is inductively coupled to the dental splint, whereby a rechargeable energy store of the dental splint can be charged.

13. A method for manufacturing a dental splint according to any one of the preceding claims, comprising
providing
- at least one base unit (13), in particular having dental splint plates which are irreversibly connected to one other, which are in particular milled or laser cut from a monolithic block, which are darned from a material, which are cast from liquid plastic, which are produced in a polymerization process, an injection molding process, a deep drawing or laser sintering process, in a rapid prototyping process, a 3D printing process, a CAD/CAM process or a wax process and/or which consist of, for example, thermoplastic and/or elastomeric material, in particular TPE material;
- at least one power supply unit (26), in particular comprising a rechargeable energy store;
- at least one signal generator (30); and
- at least one pressure sensor (16), which is operatively connected to the power supply unit (26) and to the signal generator (30), and
positioning of the pressure sensor (16) such that it can detect both occlusion forces acting on it and protrusion forces.

14. A method according to claim 13,
**characterized in that**
in order to maintain the structure of the pressure sensor (16), a spacer is temporarily introduced into the pressure sensor (16) during the manufacture of the dental splint.

## Revendications

1. Gouttière dentaire, comprenant
- une unité de base (13), en particulier avec des plaques de gouttière dentaire reliées de manière irréversible les unes aux autres,
- au moins une unité d'alimentation en énergie (26),
- au moins un générateur de signaux (30), et
- au moins un capteur de pression (16) qui est en liaison active avec l'unité d'alimentation en énergie (26) et avec le générateur de signaux (30), et qui inclut un moyen de contact avec au moins un contact de commutation électrique,
dans laquelle le capteur de pression (16) est réalisé de telle façon que celui-ci peut détecter aussi bien des forces d'occlusion que des forces de protrusion agissant sur lui-même,
**caractérisée en ce que**
le moyen de contact inclut au moins un tuyau de contact (16) qui inclut en section transversale au moins quatre segments (18) en matériau conducteur (20) et en matériau non conducteur (22) en alternance en direction périphérique.

2. Gouttière dentaire selon la revendication 1,
**caractérisée en ce que** le matériau est une matière plastique.

3. Gouttière dentaire selon la revendication 1 ou 2,
**caractérisée en ce que** les segments (18) du tuyau de contact (16) s'étendent sensiblement en continu en direction longitudinale.

4. Gouttière dentaire selon l'une des revendications 1 à 3,
**caractérisée en ce que** le tuyau de contact (16) est creux à l'intérieur.

5. Gouttière dentaire selon l'une des revendications 1 à 4,
**caractérisée en ce que** le tuyau de contact (16) inclut dans son extension longitudinale plusieurs tronçons, dans lesquels au moins un premier tronçon, qui est en particulier agencé derrière et/ou au-dessous des incisives antérieures dans la situation mise en place de la gouttière dentaire, est réalisé de manière retournée par rapport à au moins un second tronçon qui est agencé, en particulier dans la situation mise en place de la gouttière dentaire, sur les canines, sur les molaires antérieures et/ou sur les molaires postérieures.

6. Gouttière dentaire selon la revendication 5,
**caractérisée en ce qu'**au moins une partie du matériau conducteur (20) dans le premier tronçon est agencée sur les deux côtés d'un plan frontal, et **en ce qu'**au moins une partie du matériau conducteur (20) dans le second tronçon est agencée sur les deux côtés d'un plan transversal.

7. Gouttière dentaire selon l'une des revendications précédentes,
**caractérisée en ce que** le capteur de pression (16) inclut au moins deux capteurs, qui sont orientés sensiblement perpendiculairement l'un à l'autre.

8. Gouttière dentaire selon l'une des revendications précédentes,
**caractérisée en ce que** le générateur de signaux (30) et l'unité d'alimentation en énergie (26), ainsi que le cas échéant une unité de mémoire, d'émission et/ou d'évaluation, font partie intégrante d'un module.

9. Gouttière dentaire selon la revendication 8,
**caractérisée en ce que** le module forme une partie de l'unité de base (13), ou forme l'unité de base (13) elle-même.

10. Gouttière dentaire selon l'une des revendications précédentes,
**caractérisée en ce qu'**une unité centrale (14), qui inclut le générateur de signaux (30) et l'unité d'alimentation en énergie (26), ainsi que le cas échéant une unité de mémoire, d'émission et/ou d'évaluation, est entourée au moins partiellement par une feuille de séparation.

11. Gouttière dentaire selon l'une des revendications précédentes,
**caractérisée en ce que** celle-ci est formée entièrement ou partiellement d'au moins un polymère thermoplastique ou d'au moins un élastomère.

12. Station de chargement (76) pour une gouttière dentaire selon l'une des revendications précédentes, qui est réalisée sous forme de réceptacle de rangement pour la gouttière dentaire, ladite station de chargement (76) étant couplée de manière inductive avec la gouttière dentaire, de sorte qu'un accumulateur d'énergie rechargeable de la gouttière dentaire est susceptible d'être chargé grâce à celle-ci.

13. Procédé pour fabriquer une gouttière dentaire selon l'une des revendications précédentes, incluant
la mise à disposition
- d'au moins une unité de base (13), en particulier avec des plaques de gouttière dentaire reliées de manière irréversible les unes aux autres, qui sont en particulier réalisées par fraisage ou par usinage au laser à partir d'un bloc monolithique, qui sont bourrées à partir d'un matériau, qui sont coulées à partir d'une matière plastique liquide, qui sont fabriquées dans un procédé de polymérisation, un procédé de coulée par injection, un procédé d'emboutissage ou un procédé de frittage au laser, un procédé de réalisation de prototypes de type "Rapid", un procédé d'impression en trois dimensions, un procédé de conception et d'usinage assisté à l'ordinateur, ou un procédé de croissance, et/ou sont constitués par exemple d'une matière thermoplastique et/ou élastomère, en particulier d'une matière TPE,
- d'au moins une unité d'alimentation en énergie (26) incluant en particulier un accumulateur d'énergie rechargeable,
- d'au moins un générateur de signaux (30), et
- d'au moins un capteur de pression (16), qui est en liaison active avec l'unité d'alimentation en énergie (26) et avec le générateur de signaux (30), et
le positionnement du capteur de pression (16) de telle façon que celui-ci peut détecter aussi bien des forces d'occlusion que les forces de protrusion agissant sur lui-même.

14. Procédé selon la revendication 13,
**caractérisé en ce que**, pour obtenir la structure du capteur de pression (16) pendant la fabrication de la gouttière dentaire, on introduit provisoirement un élément d'espacement dans le capteur de pression (16).
